# EUROPEAN PATENT APPLICATION

(11) **EP 4 764 453 A1**
(43) Date of publication of application: **24.06.2026**
(21) Application number: 25211925.0
(22) Date of filing: 29.10.2025
(51) Int. Cl.: G01N 19/02, B65H 18/14, B65H 19/22, G01N 33/34

(54) **FRICTION MEASURING SYSTEM AND A FRICTION MEASURING METHOD FOR MEASUREMENT OF FRICTION OF A FIBER WEB IN CONNECTION WITH AN UNWINDER OF A SLITTER-WINDER**

(30) Priority: 19.12.2024 FI 20246496
(71) Applicant: Valmet Technologies Oy, 02150 Espoo (FI)
(72) Inventor: KÄÄRIÄINEN, Marko, 01370 Vantaa (FI); FÖHR, Heikki, 01370 Vantaa (FI); HAAPANEN, Jaakko, 01370 Vantaa (FI); RÄMÄ, Mikko, 01370 Vantaa (FI); SOINI, Kalle, 01370 Vantaa (FI)
(74) Representative: Berggren Oy

(57) **Abstract**

The invention relates to a friction measuring system (10) for measurement of friction of a fiber web (W) in connection with an unwinder of a slitter-winder, which unwinder is configured to unwound the fiber web (W), which fiber web (W) is configured to be run forward over at least one roll (12), which roll (12) is configured to support the run of the fiber web (W). The friction measuring system (10) comprises a friction measuring device (20), which friction measuring device (20) comprises a sample holding part (27) for holding a sample (15) cut from the fiber web (W). The friction measuring system (10) further comprises movable support elements (30-33) to support and transfer the friction measuring device (20), that the friction measuring device (20) is configured to press the sample (15) against surface of the fiber web (W) to measure the friction between the surface of the sample (15) and the surface of the fiber web (W) and a measurement movement between surfaces of the sample (15) and the fiber web (W) is provided by moving the sample (15) by the friction measuring device (20) and/or by moving the fiber web (W).

## Description

### Technical field

The invention relates generally to production of fiber webs and winding of fiber webs, especially by a winder of a slitter-winder for fiber webs. The invention relates to a friction measuring system according to the preamble part of the independent friction measuring system claim and to a friction measuring method according to the preamble part of the independent friction measuring method claim.

### Background

As known, fiber webs, such as paper or board webs, are manufactured in machines together forming a fiber web manufacturing line, which may be hundreds of meters long. Modern paper machines may produce more than 450,000 tons of paper per year. The speed of a paper machine may exceed 2000 m/min and the width of a paper web may be more than 11 meters.

As known from the prior art in fiber web producing processes typically comprise an assembly formed by a number of apparatuses arranged consecutively in the process line. A typical production and treatment line comprises a head box, a wire section and a press section as well as a subsequent drying section and a reeler or a winder. The production and treatment line can further comprise finishing devices, for example a sizer and /or a calender and / or a coater. The production and treatment line also comprises typically at least one slitter-winder for forming customer rolls as well as a roll packaging apparatus.

In fiber web production lines, manufacturing operates as a continuous process. The finished fiber web being output from the machine is wound with a reeler or a winder around a reeling shaft, i.e. a reel spool, into a parent roll (a machine roll), the diameter of which may be more than 5 meters and which may weigh more than 160 tons. The purpose of the reeling is to transfer the fiber web from its planar manufacturing form into a form in which it can be handled more easily. At the reeler, which is located in the main production line, the continuous process of the production line is interrupted for the first time, after which the process continues in stages. Every attempt is made to interlink these stages as smoothly as possible so that the work already performed would not be wasted. Reelers are thus used in fiber web production for reeling the fiber web coming from the fiber web production line into a parent roll, but reelers are also used to reel fiber web coming from a coating machine, a calender or a corresponding finishing device.

The fiber web wound in the reel-up onto the parent roll is full-width so it must be slit in longitudinal direction into partial webs with suitable width and the partial webs are wound to partial fiber web rolls (customer rolls) with suitable length of fiber web and/or of suitable diameter for the customers. The slitting and winding take place as known from prior art in an appropriate separate machine i.e. in a slitter-winder. Slitter-winders comprise an unwinder, in which the parent roll is unwound, a slitting section, in which from the parent roll unwound fiber web is slitted, i.e. cut in longitudinal direction, to partial webs with desired widths, and a winding section for winding the customer rolls. In winding sections of slitter-winders winding devices of different types are employed, for example multistation winders and two-drum winders. As known in the prior art, also winding with a shaft without cores or together with cores can be employed in the winding of a fiber web into customer rolls on a slitter-winder. On slitter-winders of the multistation winder type, the fiber web is guided from the unwinding via guide rolls to the slitting section where the fiber web is slit into partial webs which are further guided either from above or from below to the winding roll/rolls of the winding stations to be wound up onto cores into customer rolls. Adjacent partial webs are wound up on different sides of the winding roll / on different winding rolls. Multistation winders have one to three winding rolls and in them each partial web is wound to a partial web roll in its own winding station. During winding a winding nip is formed between the winding roll and the partial web rolls to be wound. In the two-drum winders the partial webs are wound around winding cores supported by two winding drums to customer rolls via a nip between the winding drums and the customer being formed. In the two-drum winders also a belt arrangement i.e. a so-called set of belt rolls with belt loop or belt loops located around two guide rolls can be used as the winding drum. During winding a winding nip is formed between the winding drums and the customer rolls wound.

As known from the prior art, in the slitter-winder the parent roll is unwound in an unwinding section in at least one unwinder, the full-width fiber web is slit on the slitting section into several narrower partial fiber webs (partial webs), which are wound up on a winding section around winding cores into customer rolls. In the slitter-winder when the customer rolls reach desired length and diameter, a set change is performed, in which the partial webs are cut in cross-direction. Tails of the partial webs i.e. trailing ends of the cut partial webs are attached onto the surface of the fiber webs on the surface of customer rolls in the set change by applying onto the surface of the fiber web running towards the customer rolls adhesive, which then attaches the tails onto the surface of the customer rolls. When the customer rolls are completed, in the slitter-winder in the set change the complete customer rolls i.e. the so-called set is removed from the winder and new cores for winding the next set of the customer rolls are set to the winder, after which the process is continued with the winding of the next set of the customer rolls. These stages are repeated periodically until paper runs out of the parent roll in the unwinder, whereby a parent roll change is performed and the operation starts again as the unwinding of a next parent roll.

Vibration phenomenon may cause problems in winders. The vibration causes winding reject, mechanical wear of the parts and element of the slitter-winder, even loosening of the customer rolls from the winder as well as decrease of winding capacity as the running speed has to be lowered. The vibration phenomenon thus, often limits the maximum speed utilizable in the winding and therefore decreases capacity of the slitter-winder. In the worst case, the vibration phenomenon can cause the out-throwing of the set of the customer rolls from the winder. Additionally, web breaks may occur due to the vibration phenomenon. The web breaks may also be caused by weakness of the fiber web of by defects in the fiber web.

In utility model publication CN222166843U is disclosed a friction measuring system for measurement of friction of a web. The web is configured to be run forward over at least one roll, which roll is configured to support the run of the web. The friction measuring system comprises a friction measuring device, which friction measuring device comprises a sample holding part for holding a material sample. The friction measuring system further comprises movable support elements to support and transfer the friction measuring device and the friction measuring device is configured to press the sample against surface of the web to measure the friction between the surface of the sample and the surface of the web. A measurement movement between surfaces of the sample and the web is provided by moving the sample by the friction measuring device.

In patent publication US5490410 is disclosed a friction measuring system for measurement of friction of a fiber web.

An object of the present invention is to create a friction measuring system and a friction measuring method, in which the above problems and disadvantages are eliminated or at least minimized.

A particular object of the present invention is to an improved friction measuring system and an improved friction measuring method, in which contamination of samples is eliminated or at least minimized and thus, reliable results are achieved.

### Summary

To achieve the above-mentioned objects and those which come out later, the friction measuring system according to the invention is mainly characterized by what is presented in the characterizing part of the independent friction measuring system claim and the friction measuring method according to the invention is mainly characterized by what is presented in the characterizing part of the independent friction measuring method claim. Advantageous features and embodiments of the invention are defined in the claims.

According to the invention the friction measuring system for measurement of friction of a fiber web in connection with an unwinder of a slitter-winder, which unwinder is configured to unwound the fiber web, which fiber web is configured to be run forward over at least one roll, which roll is configured to support the run of the fiber web, wherein the friction measuring system comprises a friction measuring device, which friction measuring device comprises a sample holding part for holding a sample cut from the fiber web, wherein the friction measuring system further comprises movable support elements to support and transfer the friction measuring device, wherein the friction measuring device is configured to press the sample against surface of the fiber web to measure the friction between the surface of the sample and the surface of the fiber web and wherein a measurement movement between surfaces of the sample and the fiber web is provided by moving the sample by the friction measuring device and/or by moving the fiber web.

According to the invention the friction measuring device comprises a sample cutting tool for cutting the sample from the fiber web.

Advantageously, the fiber web is supported during the friction measurement. According to an advantageous feature of the invention the fiber web is supported by the roll during the friction measurement by the friction measuring device. Alternatively advantageously, the fiber web is supported by the parent roll, from which it is unwound. Alternatively advantageously, a support element is provided at the friction measurement location.

According to an advantageous feature of the invention the friction measuring device has transfer movement/-s to transfer the sample to a measurement location and a measurement movement in the measurement location and that the sample holding part of the friction measuring device is configured to hold the sample during the transfer movement/-s to the measurement location and during the measurement movement.

According to an advantageous feature of the invention the friction measuring system is an automatically operating friction measuring system.

According to an advantageous feature of the invention the friction measuring device is configured to press bottom surface of the sample against top surface of the fiber web.

According to an advantageous feature of the invention the sample and the fiber web, onto which surface the sample is configured to be pressed during the friction measurement, are from the same fiber web roll unwound in the unwinder.

According to an advantageous feature of the invention the friction measuring device is configured to move the sample from level of the surface of the fiber web after cutting the sample by the sample cutting tool above the surface of the fiber web by the movable support elements.

According to the invention the friction measuring method for measurement of friction of a fiber web in connection with an unwinder of a slitter-winder, which unwinder unwinds the fiber web from a fiber web roll, which unwound fiber web runs forward over at least one roll, which roll supports the run of the fiber web, wherein a sample is cut from the fiber web, the sample is moved to a measurement location, the sample is pressed against the surface of the unwound fiber web and a measurement movement is caused between the surface of the sample and the surface of the fiber web to measure the friction between the surface of the sample and the surface of the fiber web for conducting a friction measurement.

According to an advantageous feature of the invention in the method the measurement movement is caused by moving the friction measurement device, which is holding the sample, in cross direction of the fiber web.

Advantageously, in the method the measurement is performed at a location, where the fiber web is supported. According to an advantageous feature of the invention in the method the measurement movement is performed at the location, where the roll supports the fiber web. Alternatively advantageously, the fiber web is supported by the parent roll, from which it is unwound, or the fiber web is supported by a support element located at the measurement location.

According to an advantageous feature of the invention in the method the sample is moved from level of the surface of the fiber web (W) after cutting the sample above the surface of the fiber web for the friction measurement.

According to an advantageous feature of the invention in the method friction measurement is conducted over the whole substantial width direction of the fiber web.

According to an advantageous feature of the invention from the friction measurement friction measurement results are received and the friction measurement results are transmitted to a control system of the fiber web production line, in connection of which the unwinder of the slitter-winder is located, and/or to a part thereof to provide friction information for controlling the fiber web production.

According to an advantageous feature of the invention the friction measurement is performed directly after the fiber web roll rotation is stopped in the unwinder for a parent roll change or for a set change while the fiber web is stopped and the slitting and winding sections of the slitter-winder are stopped.

According to an advantageous feature of the invention in the method bottom surface of the sample is pressed against top surface of the fiber web during the measurement movement.

It has been surprisingly noted that in winding of fiber webs friction between the fiber web layers effects to vibration phenomenon in winders. Typically, the friction properties of a fiber web have not been measured in connection with fiber web production lines. It is known that in some rare cases friction of the fiber web has been measured, but this has been fully manual operation, in which to measure friction of the surface of the fiber web, a sample / sample from the fiber web has been manually taken and then, the friction is measured in a laboratory. Friction measurement is very sensitive even in laboratory conditions, because contamination of samples, even a small stain of outside material on the surface of a sample, for example an oily fingerprint of a measuring person, compromises results of friction measurement.

The present invention provides a novel and inventive friction measurement in connection with an unwinder of a slitter-winder. By the friction measuring system and the friction measuring system according to the invention the friction of a fiber web is found out in respect of a fiber web roll unwound in the unwinder and thus, the friction measurement results can be used in connection with the winding of the fiber web to prevent vibration. The novel and inventive friction measurement by the friction measuring system and the friction measuring system according to the invention the fouling of the sample is prevented as well as the measurement can be conducted directly after the sample has been cut.

Advantageously, the friction measurement is conducted for several successive fiber web rolls, more advantageously for each fiber web roll, unwound in the unwinder and thus, results of the friction and its variation is received in view of the produced fiber web.

Advantageously, the friction measurement is conducted over the whole substantial width of the fiber web and thus, information in regard of friction variation in cross direction of the fiber web is achieved.

Advantageously, the friction measurement is automatized. This provides for saving in operating personnel costs, human errors can be avoided etc.

Further advantageously, the friction measurement results received are transmitted to a control system of a fiber web production line, in connection of which the unwinder of the slitter-winder is located, and/or to a part thereof to provide friction information for controlling the fiber web production such, that friction properties of the fiber web are as desired, i.e. not causing vibration. Furthermore advantageously, the friction measurement results received can be transmitted to a control system of a pulp production and/or a pulp treatment system providing pulp mass for the fiber web production line, in connection of which the unwinder of the slitter-winder is located, and/or to a part thereof to provide friction information for controlling the pulp production and/or treatment such, that friction properties of the pulp mass are as desired, i.e. not causing vibration.

According to an advantageous aspect of the invention in the friction measuring system and the friction measuring system the sample and the fiber web against which the sample is pressed for the friction measurement are of the same fiber web roll. Advantageously, a bottom side of the sample is pressed against a top side of the fiber web or vice versa. Friction varies also depending on the side of the fiber web, especially for example is such grades of fiber webs, which are coated one-sidedly. Thus, in view of using the friction measurement results in connection with winding, it is important that the friction measurement results correspond to the winding layers.

According to an advantageous aspect of the invention in the friction measurement by the friction measuring system and the friction measuring system, the sample of the fiber web is pressed against the surface of the fiber web at a known, selected force needed to move the sample, i.e. the force effecting during movement of the sample, is measured by a force sensor and thus, based on the measured force the friction force is defined.

Advantageously, in the friction measurement the sample is moved from the fiber web level above the fiber web for the friction measurement.

According to an advantageous aspect of the invention the friction measurement by the friction measuring system and the friction measuring system according to the invention is performed directly after the fiber web roll rotation is stopped in the unwinder of the slitter-winder for a parent roll change or for a set change, while the fiber web is stopped and the slitting and winding sections are stopped. This provides that the friction measurement does not disturb the fiber web production or slows down the sequence or decreases production.

According to an advantageous aspect of the invention the sample is cut from the fiber web by a sample cutting tool. Advantageously, the friction measuring device comprises the sample cutting tool. The sample cutting tool can also be a separate device of the friction measuring system, which friction measuring system then also has means to transfer the cut sample to holding of the sample holding part of the friction measuring device or the sample is transferred manually. Furthermore, the sample can be cut manually from the fiber web and then be manually placed to holding of the sample holding part of the friction measuring device.

According to an advantageous aspect of the invention the sample is cut from an edge of the fiber web. Typically, in slitting section the edges of the fiber web are cut and rejected. Thus, the part of the edge with the cut opening from the sample cutting does not cause production losses in the winding, as this part would have in any case guided to the reject handling.

According to an advantageous aspect of the invention the sample for the friction measurement is taken from the end part of the fiber web roll, i.e. from the fiber web unwound from the bottom layers of the fiber web roll.

### Brief description of the drawings

Aspects of the invention, however, together with additional objects and advantages thereof, will be best understood from the following description of some example embodiments when read in connection with the accompanying drawings and in the following the invention is described in more detail referring to the accompanying drawing, in which
In figures 1A-1C is schematically shown an advantageous example of the friction measuring system and the friction measuring method according to the invention seen from above,
In figures 2A-2B is schematically shown the example of figures 1A-1C as a side view and
In figure 3 is schematically shown an advantageous example of a friction measuring device for the friction measuring system and the friction measuring method according to the invention as a 3D view.

### Detailed description

In the following description same reference signs designate for similar components unless otherwise mentioned and it should be understood that the examples are susceptible of modification in order to adapt to different usages and conditions within the frames of the invention. Repetition of some reference signs may have been omitted in the figures for clarity reasons. In some of the figures an arrow has been used to indicate rotation direction of the respective main element of the construction.

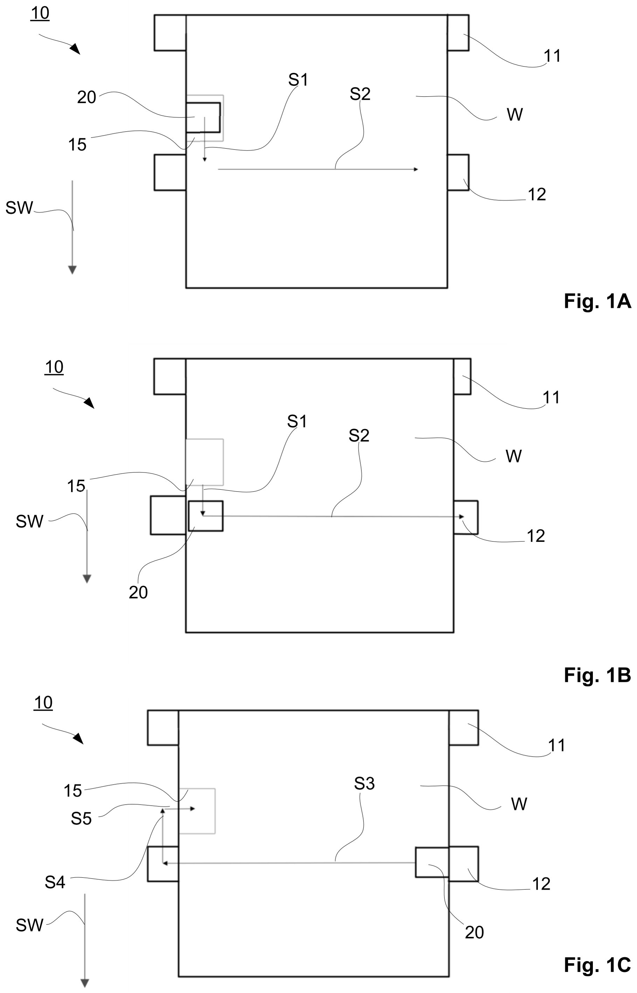

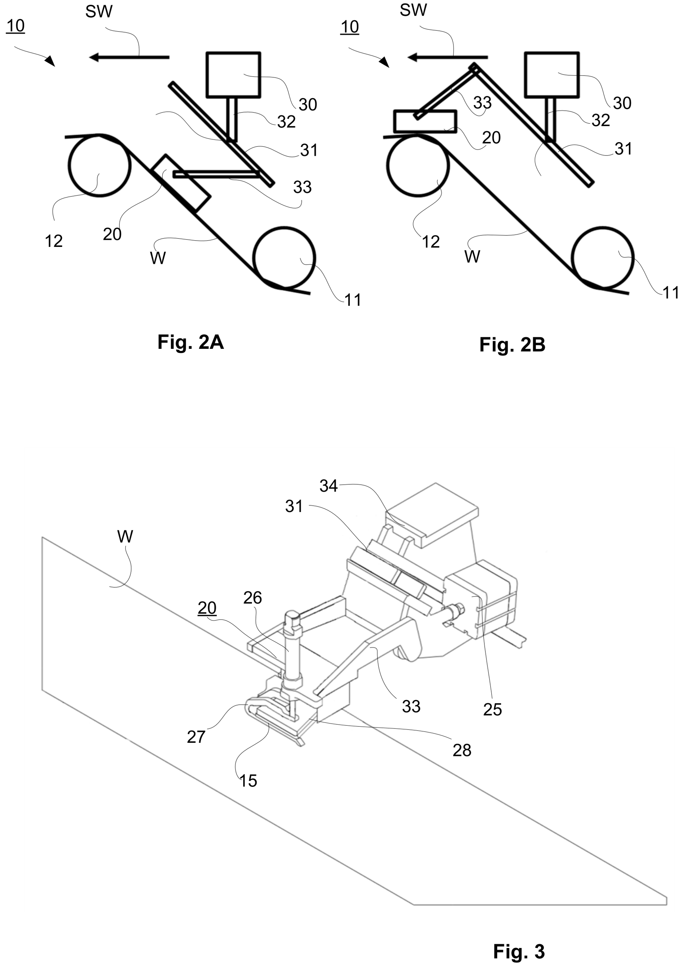

In figures 1A-1C and 2A-2B is shown an example of the friction measuring system 10 and the friction measuring method. In this example friction of a fiber web W is measured in connection with an unwinder of a slitter-winder. Fiber web W is unwound in the unwinder from a fiber web roll 11 and guided forward towards a slitting section (not shown) of the slitter-winder as indicated by arrow SW. During its run the fiber web W passes on a roll 12, which supports the run of the fiber web W. In order to measure friction of the fiber web W the friction measuring system 10 comprises a friction measuring device 20, which has a sample cutting tool 28 (fig. 3) for cutting a sample 15 from an edge of the fiber web W, and a sample holding part 27 (fig. 3) to hold the sample 15 after cutting thereof and during its transfer movement/-s S1 to a measurement location and during measurement movement S2. The friction measuring device 20 has also return movements S3, S4, S5 for returning the friction measuring device 20 to the sample cutting location for next sample cutting. The sample cutting tool 28 advantageously removes the sample from the fiber web by die cutting, knife cutting, rotating blade cutting or by perforation cutting. The sample holding part 27 is advantageously a suction holder, for example provided with suction cups, a mechanical holder or an adhesion holder. The friction measuring system 10 comprises movable support elements 30-33 to support and transfer the friction measuring device 20. The movable support elements 30-33 comprise a cross direction linear guide 30, a machine direction linear guide 31, a first supporting mechanism 32 and a second supporting mechanism 33. The friction measuring device 20 is connected by the second supporting mechanism 33 movably in machine direction on the machine direction linear guide 31. For movement in cross direction of the friction measuring device 20 the machine direction linear guide 31 is movably in cross direction connected at a connection point 34 by the first supporting mechanism 32 to the cross direction linear guide 30.

The friction fiber web W is advantageously measured during a parent roll change of the unwinder or during a set change of the slitter-winder, when the unwinder is stopped and the slitting and winding is stopped. During the transfer movement/-s S1 the sample 15 is at a distance from the surface of the fiber web W, i.e. the sample 15 does not touch the surface of the fiber web W. During the measurement movement S2 the sample 15 is pressed against the surface of the fiber web W at a known, selected force and force needed to move the sample, i.e. the force effecting during movement of the sample, is measured by a force sensor and thus, based on the measured force the friction force is defined. During the return movement S3 the sample is held by the friction measuring device 20. Before the final return movements S4, S5 back to the sample taking location the sample 15 has been released from the friction measuring device 20.

At first the friction measuring device 20 of the friction measuring system 10 is moved to a sample cutting location, which is shown in figures 1A and 2A, and a part of an edge of the fiber web W is crapped by the sample holding part 27 (fig. 3) and a sample 15 is cut from the fiber web W around the friction measuring device 20 by the sample cutting tool 27 (fig. 3).

After the sample cutting the friction measuring device 20 with the sample 15 is transferred to the friction measurement location by a machine directional movement S1 to a location above the roll 12 supporting the fiber web W and then by during a cross directional movement i.e. the measurement movement S2 the friction measuring device 20 is moved to a location at opposite edge of the fiber web W, as shown in figures 1B and 2B. During the measurement movement S2 the sample 15 is pressed against the surface of the fiber web W at a known, selected force and force needed to move the sample, i.e. the force effecting during movement of the sample, is measured by a force sensor and thus, based on the measured force the friction force is defined. During the measurement the fiber web W can alternatively be supported by the parent roll, from which it is unwound, or by a separate support element.

During the friction measurement the roll 12 also functions as a support for the measurement and the friction measuring device 20 presses the sample 15 against the surface of the fiber web W on the roll 12.

After the friction measurement the friction measuring device 20 lifts the sample 15 upwards from the contact with the surface of the fiber web. The friction measuring device 20 with the sample 15 is moved back to the other edge of the fiber web W during the return movement S3, as shown in figures 1C and 2B.

It should be noted that also during the return movement S3 the friction measuring device 20 can be positioned to a measurement position, i.e. the sample 15 pressed against the surface of the fiber web W. Further, the measurement movement S2 can be a transfer movement and the return movement S3 the measurement movement.

When the friction measurement has been done and the friction measuring device 20 has been returned from the opposite edge of the fiber web, the sample 15 is released from the friction measuring device 20 and the friction measuring device 20 is returned back to the sample cutting position by final return movements S4, S5, as indicated in figure 1C by arrows S4, S5.

In figure 3 is shown an example of a friction measuring device 20 for the friction measuring system 10 and the friction measuring method. In this example the friction measuring device 20 comprises the sample cutting tool 28 and the sample holding part 27 operated by an actuator 26. The friction measuring device 20 also comprises an actuator 25, for example a pneumatic cylinder 25, to provide movements of the friction measuring device 20. The friction measuring device 20 is connected by the second supporting mechanism 33 movably in machine direction on the machine direction linear guide 31. For movement in cross direction of the friction measuring device 20 the machine direction linear guide 31 is movably in cross direction connected at a connection point 34 by the first supporting mechanism 32 (figs. 2A-2B) to the cross direction linear guide 30 (figs. 2A-2B).

In the description in the foregoing, although some functions have been described with reference to certain features, those functions may be performable by other features whether described or not. Although features have been described with reference to certain embodiments or examples, those features may also be present in other embodiments or examples whether described or not. Above the invention has been described by referring to some advantageous examples only to which the invention is not to be narrowly limited. Many modifications and alterations are possible within the invention as defined in the following claims.

## Claims

1. Friction measuring system (10) for measurement of friction of a fiber web (W) in connection with an unwinder of a slitter-winder, which unwinder is configured to unwound the fiber web (W), which fiber web (W) is configured to be run forward over at least one roll (12), which roll (12) is configured to support the run of the fiber web (W), which friction measuring system (10) comprises a friction measuring device (20), which friction measuring device (20) comprises a sample holding part (27) for holding a sample (15) cut from the fiber web (W), which the friction measuring system (10) further comprises movable support elements (30-33) to support and transfer the friction measuring device (20), which the friction measuring device (20) is configured to press the sample (15) against surface of the fiber web (W) to measure the friction between the surface of the sample (15) and the surface of the fiber web (W) and wherein a measurement movement between surfaces of the sample (15) and the fiber web (W) is provided by moving the sample (15) by the friction measuring device (20) and/or by moving the fiber web (W), **characterized in, that** the friction measuring device (20) comprises a sample cutting tool (28) for cutting the sample (15) from the fiber web (W).

2. Friction measuring system according to claim 1, **characterized in, that** the fiber web (W) is supported by the roll (12) during the friction measurement by the friction measuring device (20).

3. Friction measuring system according to claim 1 or 2, **characterized in, that** the friction measuring device (20) has transfer movement/-s (S1) to transfer the sample (15) to a measurement location and a measurement movement (S2) in the measurement location and that the sample holding part (27) of the friction measuring device (20) is configured to hold the sample during the transfer movement/-s (S1) to the measurement location and during the measurement movement (S2).

4. Friction measuring system according to any of claims 1 - 3, **characterized in, that** the friction measuring system is an automatically operating friction measuring system.

5. Friction measuring system according to any of claims 1 - 4, **characterized in, that** the friction measuring device (20) is configured to press bottom surface of the sample (15) against top surface of the fiber web (W).

6. Friction measuring system according to any of claims 1 - 5, **characterized in, that** the sample (15) and the fiber web (W), onto which surface the sample (15) is configured to be pressed during the friction measurement, are from the same fiber web roll unwound in the unwinder.

7. Friction measuring system according to any of claims 1 - 6, **characterized in, that** the friction measuring device (20) is configured to move the sample (15) from level of the surface of the fiber web (W) after cutting the sample (15) by the sample cutting tool (28) above the surface of the fiber web (W) by the movable support elements (30-33).

8. Friction measuring method for measurement of friction of a fiber web (W) in connection with an unwinder of a slitter-winder, which unwinder unwinds the fiber web (W) from a fiber web roll (11), which unwound fiber web (W) runs forward over at least one roll (12), which roll (12) supports the run of the fiber web (W), **characterized in, that** in the method a sample (15) is cut from the fiber web (W), the sample (15) is moved to a measurement location, the sample (15) is pressed against the surface of the unwound fiber web (W) and a measurement movement (S2) is caused between the surface of the sample (15) and the surface of the fiber web (W) to measure the friction between the surface of the sample (15) and the surface of the fiber web (W) for conducting a friction measurement.

9. Friction measuring method according to claim 8, **characterized in, that** in the method the measurement movement (S2) is caused by moving the friction measurement device (20), which is holding the sample (15), in cross direction of the fiber web (W).

10. Friction measuring method according to claim 8 or 9, **characterized in, that** in the method the measurement movement (S2) is performed at the location, where the roll (12) supports the fiber web (W).

11. Friction measuring method according to any of claims 8 - 10, **characterized in, that** in the method the sample (15) is moved from level of the surface of the fiber web (W) after cutting the sample (15) above the surface of the fiber web (W) for the friction measurement.

12. Friction measuring method according to any of claims 8 - 11, **characterized in, that** in the method friction measurement is conducted over the whole substantial width direction of the fiber web (W).

13. Friction measuring method according to any of claims 8 - 12, **characterized in, that** from the friction measurement friction measurement results are received and the friction measurement results are transmitted to a control system of the fiber web production line, in connection of which the unwinder of the slitter-winder is located, and/or to a part thereof to provide friction information for controlling the fiber web production.

14. Friction measuring method according to any of claims 8 - 13, **characterized in, that** the friction measurement is performed directly after the fiber web roll rotation is stopped in the unwinder for a parent roll change or for a set change while the fiber web is stopped and the slitting and winding sections of the slitter-winder are stopped.

15. Friction measuring method according to any of claims 8 - 14, **characterized in, that** in the method bottom surface of the sample (15) is pressed against top surface of the fiber web during the measurement movement.
